# EUROPEAN PATENT SPECIFICATION

(11) **EP 3 591 060 B1**
(45) Date of publication and mention of the grant of the patent: **24.01.2024**
(21) Application number: 19183148.6
(22) Date of filing: 28.06.2019
(51) Int. Cl.: C12N 15/87, C12N 5/00

(54) **AN ELECTROPORATION SOLUTION AND AN ELECTROPORATION PROCESS PERFORMED WITH THIS SOLUTION**
ELEKTROPORATIONSLÖSUNG UND EIN MIT DIESER LÖSUNG DURCHGEFÜHRTES ELEKTROPORATIONSVERFAHREN
SOLUTION D'ÉLECTROPORATION ET PROCÉDÉ D'ÉLECTROPORATION MIS EN UVRE AU MOYEN DE CETTE SOLUTION

(30) Priority: 04.07.2018 TR 201809546
(43) Date of publication of application: 08.01.2020
(73) Proprietor: Yeditepe Universitesi, 34755 Istanbul (TR)
(72) Inventor: SAHIN, Fikrettin, Istanbul (TR); AYDIN, Safa, 34755 Atasehir - Istanbul (TR); TASLI, Pakize Neslihan, 34755 Atasehir - Istanbul (TR)
(74) Representative: Dericioglu, E. Korhan

(56) References cited:
- EP-A1- 2 889 373
- SAFA AYDIN ET AL: "Pluronic PF68 increases transfection efficiency in electroporationof mesenchymal stem cells", TURKISH JOURNAL OF BIOLOGY, vol. 40, 2016, pages 747-754, XP055637065, TR ISSN: 1300-0152, DOI: 10.3906/biy-1503-55
- YILMAZ ET AL: "Effect of Boron and F68 Pluronic Block Copolymer Combination on Adipogenic Differentiation of Human Adipose Stem Cells", THESIS, GRADUATE SCHOOL OF NATURAL AND APPLIED SCIENCES, YEDITEPE UNIVERSITY, TURKEY, 12 September 2014 (2014-09-12), pages 1-52, XP009516192,
- RYUSUKE TACHIBANA ET AL: "Effect of Melatonin on Human Dental Papilla Cells", INTERNATIONAL JOURNAL OF MOLECULAR SCIENCES, vol. 15, no. 10, 26 September 2014 (2014-09-26), pages 17304-17317, XP055637077, DOI: 10.3390/ijms151017304
- AYSEGÜL DOGAN ET AL: "Effect of F68 on Cryopreservation of Mesenchymal Stem Cells Derived from Human Tooth Germ", APPLIED BIOCHEMISTRY AND BIOTECHNOLOGY, vol. 171, no. 7, 3 September 2013 (2013-09-03), pages 1819-1831, XP55637176, New York ISSN: 0273-2289, DOI: 10.1007/s12010-013-0472-z

## Description

### Field of the Invention

The present invention relates to an electroporation solution comprising melatonin, F68 and NaB developed for carrying out gene transfer easily and with high efficiency to cells, to which gene transfer is difficult/not difficult, by means of electroporation method. The invention is also related to a method for electroporation process carried out by using this electroporation solution.

### Background of the Invention

Electroporation is one of the techniques used in molecular biology to introduce a foreign gene or material into the cell. Electroporation is the process of opening nanometer-sized transient pores in the cell membranes by brief application of strong electrical current to the cells or tissues. With this method, the double layer phospholipid structure of the cell membrane is disrupted for a short period of time. The permeability of the cell plasma membrane increases significantly and this permeability is used to introduce some substances into the cell [1]. The permeabilized state caused by the pores formed when the electric current is applied to the cells can be used for .passage of DNA, RNA, drugs and various antibodies through the cell membrane **[2].** Gene transfer activity may vary according to factors such as cell concentration, temperature, electroporation solution, DNA concentration, current type and voltage **[3].**

As with all gene transfer methods, electroporation also has some advantages and disadvantages. The facts that it is a fast method, has a wide area of use, does not show toxic effects and is an inexpensive and efficient method are among the advantages of the electroporation method, while the cell deaths that occur when electric current is applied and physiological deformities that may result from the imbalance of the substances exiting the cell are among the disadvantages of the method.

In order to achieve long-term gene transfer, gene transfer via viral vectors is often preferred, but this technique has significant disadvantages in terms of biological safety **[4].** Chemical based gene transfer methods basically utilize cationic lipids and polymers and are simple to use, but they are insufficient to transfect mesenchymal stem cells. Furthermore, some transfection agents also have toxicity problems.

Dental pulp, which is one of the tissues in which stem cells are isolated, is a soft tissue that is surrounded by dental enamel, is in the center of the tooth and is surrounded by blood vessels and nerves. Dental pulp is a combination of ectodermic and mesodermic contents with multiple potential neural crest cells **[5].** The dental pulp contains stem cells that are involved in the formation of tooth enamel. The tooth germ and bone marrow stem cells CD44, CD73 and CD90 contain the same stem cell surface antigens. These stem cells may differentiate into muscle, cartilage and bone cells **[6] [7].**

In a study conducted in 2008, electroporation parameters were optimized for stem cells obtained from bone marrow stem cells, but either efficiency was too low or cell death was too high [8]. In a study conducted in 2009, genes were transferred to the stem cells obtained from human tooth germs by electroporation method. In this study, the number of cells receiving the gene was as low as 39%, while the cell viability after electroporation was only 69% **[3].**

International patent application no. WO2017050884, an application known in the state of the art, discloses a method developed for high level and stable gene transfer in lymphocytes. The method of the said invention enables higher gene-transfer rates and is at the same time less toxic than the conventional approach. For this purpose, plasmid DNA-encoded transposase in combination with a plasmid DNA-encoded transposable element is used.

United States patent application no. US2013122592, an application known in the state of the art, discloses a foreign gene transfer method by electroporation. Within the scope of the invention, a conventional buffer solution and a liquid medium in which targeted animal cells can be proliferated may be used. A buffer solution used for the conventional electroporation technique, such as PBS or HEPES, can be used as the buffer solution.

United States patent application no. US2008215032, an application known in the state of the art, discloses about minimizing metal toxicity during electroporation enhanced delivery of polynucleotides. It is aimed to minimize the side effects to the electroporated tissue arising from the metal contaminants released from the electrodes. In one embodiment of the invention, gold, gold alloys, or other metals that minimize the introduction of toxic amounts of the metal into electroporated tissue are used as the electrodes.

The main disadvantages of the state of the art applications can be listed as follows:
- Toxicity of the chemicals used in gene transfer to the cell,
- Failure of the cells to resist the electric current applied to the cell during electroporation,
- Low gene transfer efficiency.

### Summary of the Invention

The objective of the present invention is to provide an electroporation solution which enables gene transfer to cells, to which gene transfer is difficult/not difficult, easily and with high efficiency by means of electroporation method.

### Detailed Description of the Invention

**"An electroporation solution and method of production thereof"** developed to fulfill the objective of the present invention is illustrated in the accompanying figures, in which:

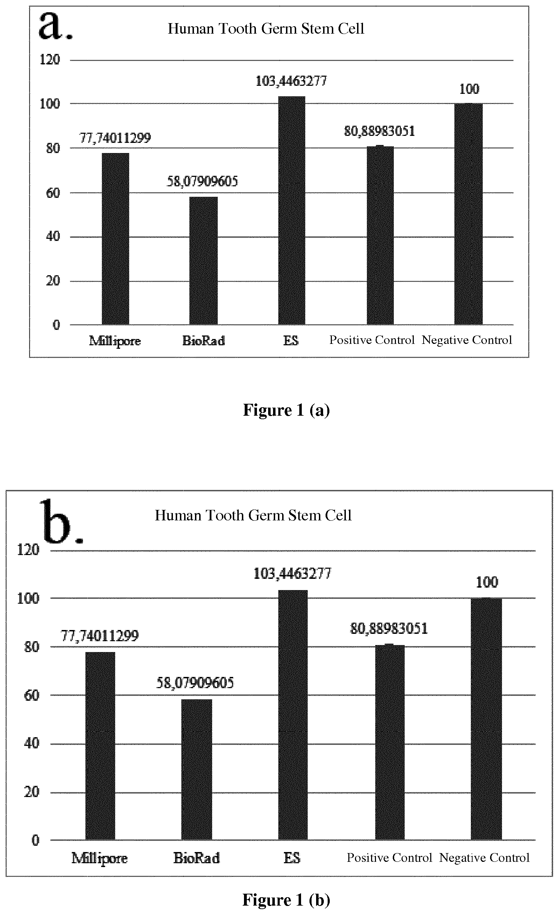

- **Figure 1**: is a graphical representation of viability of human tooth germ stem cell (a) and human bone marrow stem cell (b) in percentage 24 hours after electroporation. (ES: Electroporation Solution)

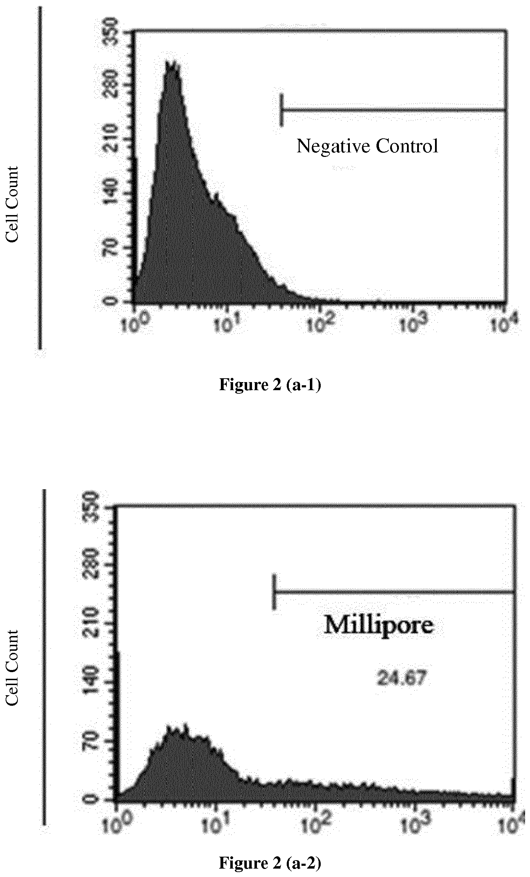

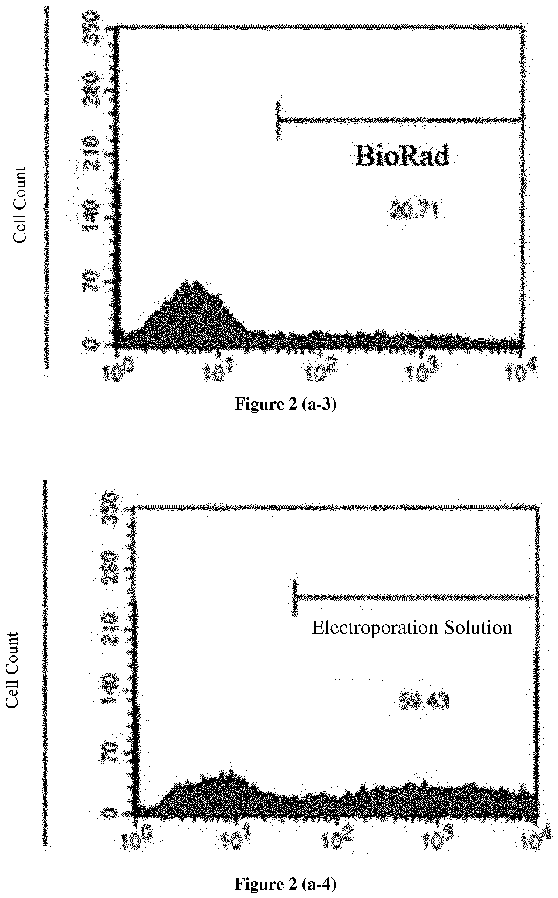

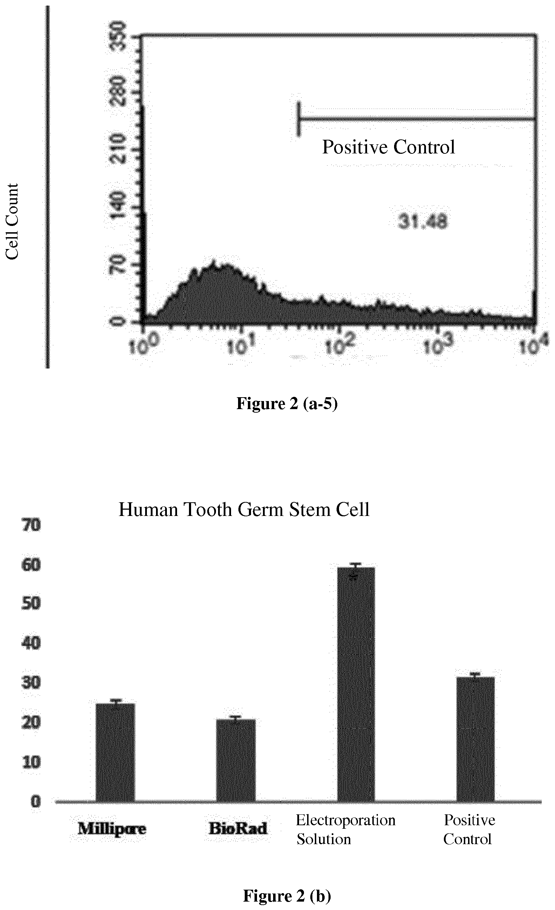

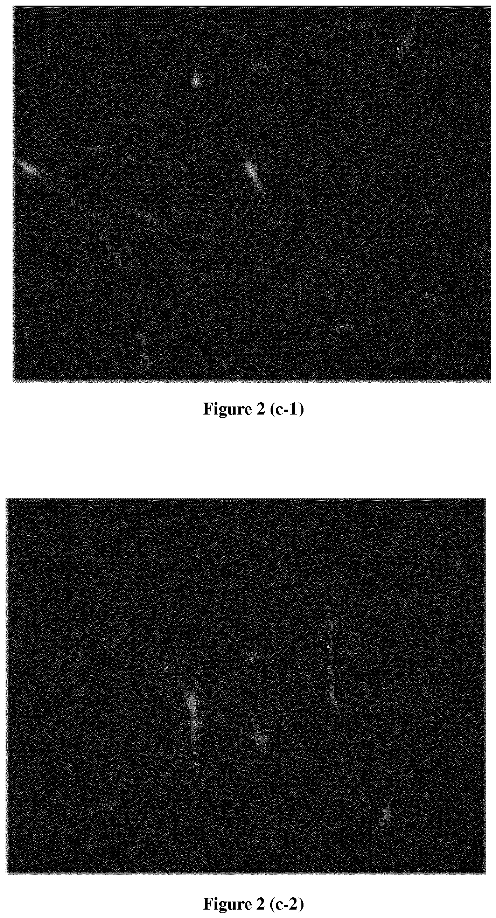

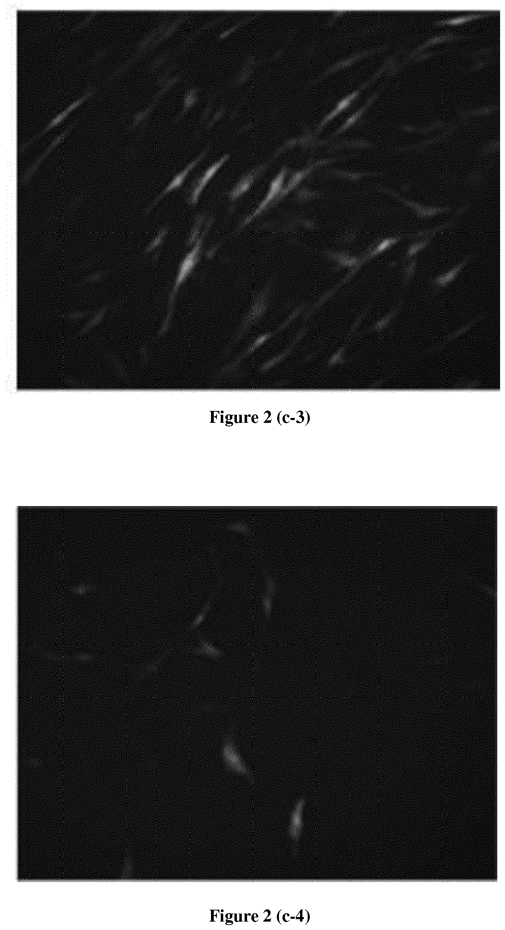

- **Figure 2**: is a representation of the data of electroporation efficiency flow cytometry (a) (1- Negative control, 2- Millipore, 3- Biorad, 4-Electroporation solution of the present invention, 5- Positive control), graphically (b) and expression of EGFP gene under fluorescent microscope 1- Millipore, 2- Biorad, 3- Electroporation solution of the present invention, 4- Positive control (c). (the cells to which PBS was applied during electroporation were indicated as the positive control, and the cells to which electroporation was not applied were indicated as the negative control).

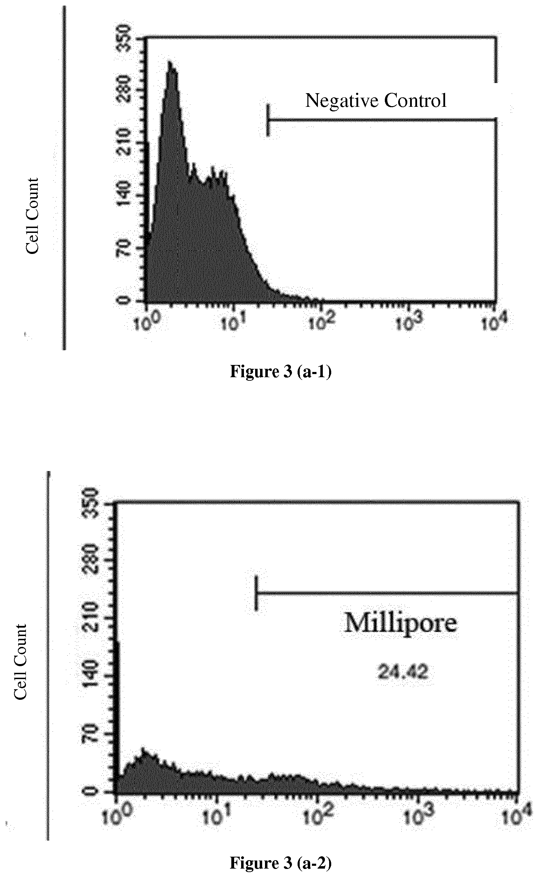

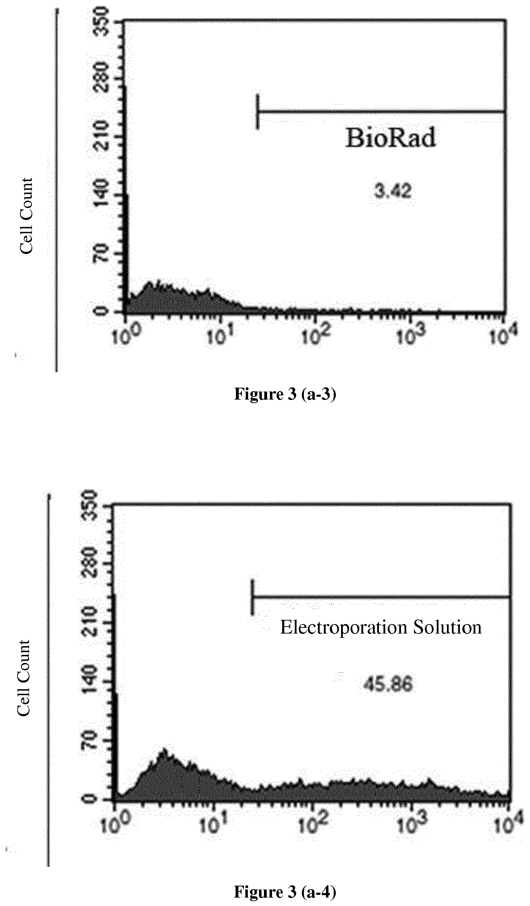

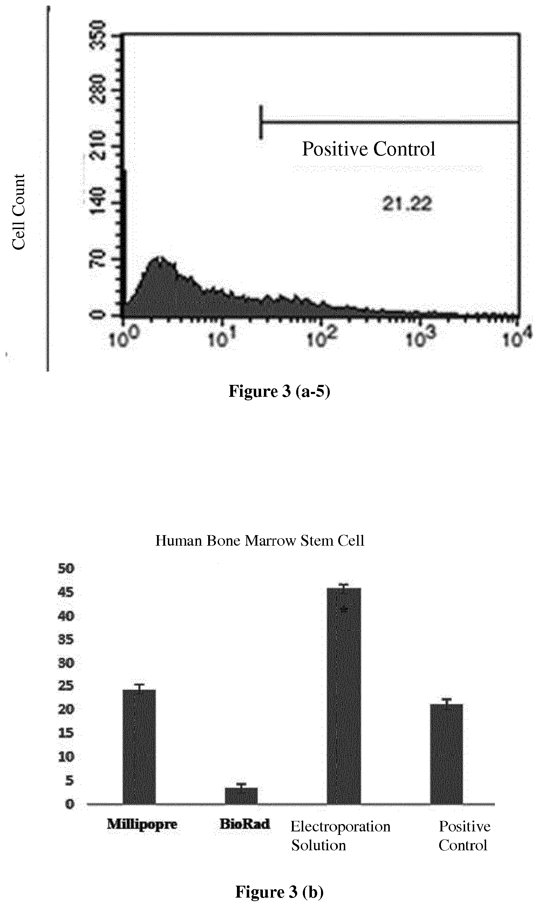

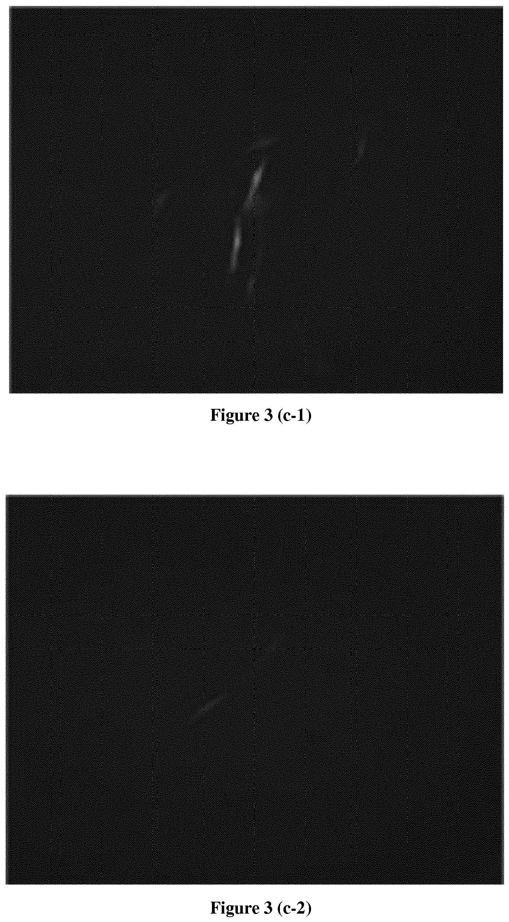

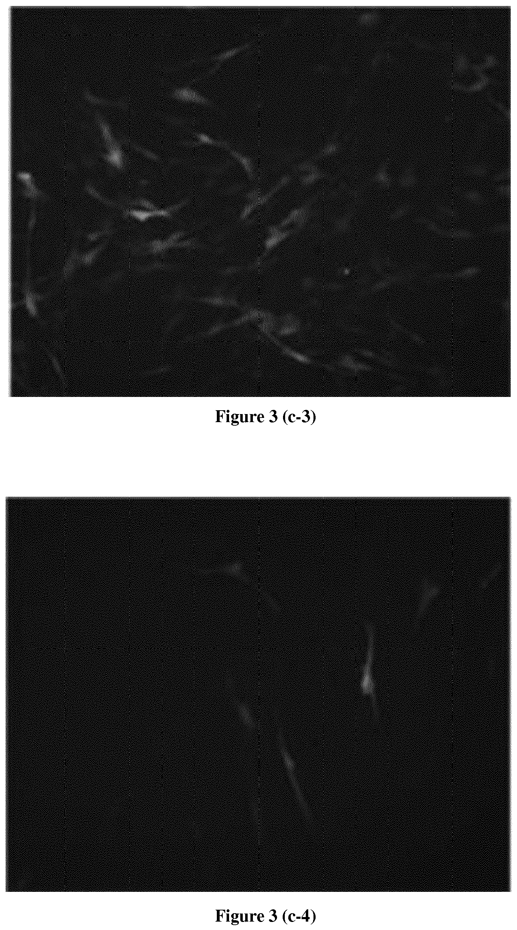

- **Figure 3**: is a representation of the data of electroporation efficiency flow cytometry (a) (1- Negative control, 2- Millipore, 3- Biorad, 4-Electroporation solution, 5- Positive control), graphically (b) and expression of EGFP gene under fluorescent microscope 1- Millipore, 2-Biorad, 3- Electroporation solution of the present invention, 4- Positive control (c). (the cells to which PBS was applied during electroporation were indicated as the positive control, and the cells to which electroporation was not applied were indicated as the negative control).

The electroporation solution of the present invention is an electroporation solution comprising Melatonin, sodium pentaborate pentahydrate (NaB) and Pluronic F68 solution, and is produced by preparing stock solutions comprising the said components and then diluting them and mixing in a medium (RPMI (Roswell Park Memorial Institute)). These stock solutions are as follows:
- Sodium pentaborate pentahydrate stock solution with a concentration of 0.1 g/ml obtained by dissolving sodium pentaborate pentahydrate (NaB) solution in a medium,
- Pluronic F68 stock solution with a concentration of 0.1 g/ml obtained by dissolving Pluronic F68 solution in cold PBS,
- Melatonin stock solution with a concentration of 10mM obtained by dissolving Melatonin solution in ethanol.

For the preparation of the electroporation solution of the present invention, 50 nM Melatonin, 5 mg/mL F68 and 20mg/mL NaB solutions, which are produced by diluting the above described stock solutions, are used.

The Electroporation process using the electroporation solution prepared according to the invention comprises the following steps:
- dissolving human tooth germ stem cells that have reached a concentration of 60 to 80 percent in trypsin-EDTA to obtain the cell solution,
- precipitating the cells by placing the cell solution into falcon tubes and centrifuging them,
- dissolving the precipitated cell pellet in a medium,
- placing EGFP plasmid in the medium and transferring the resulting solution to an electroporation vessel containing the electroporation solution,
- applying electric current to the cells within the scope of the electroporation process,
- seeding the cells into culture plates after electroporation,
- obtaining the cell culture to which gene transfer is performed.

In addition to the fact that the electroporation solution developed in accordance with the invention is not toxic to the cells in the process of gene transfer by electroporation, this electroporation solution increases cell viability and ensures high gene transfer efficiency.

It is within the advantages of the present invention that the solutions of the invention both reduce cell death and minimize substance release from the cell by ensuring cell membrane stability.

### Experimental Studies

### Preparation of the Stock Solutions

Sodium pentaborate pentahydrate stock solution with a concentration of 0.1 g/ml was prepared by dissolving sodium pentaborate pentahydrate (NaB) solution in a culture medium. A stock solution with a concentration of 0.1 g/ml was prepared by dissolving Pluronic F68 solution in cold PBS (Phosphate-Buffered Saline). Melatonin stock solution was prepared by dissolving Melatonin solution in ethanol. After the stock solution was filtered through an average of 0.2 µm filter (Sartorius AG, Göttingen, Germany) and sterilized, lower concentrations were prepared by using Dulbecco's modified Eagle's medium (Invitrogen, Carlsbad, CA).

### Pre-electroporation solution (Pre-incubation solution)

Pre-incubation solution protects the cells from getting damaged from the electric current to be generated by electroporation by feeding them prior to electroporation. 10-100 nM Melatonin and 1-10 mg/mL F68 solutions were diluted from their stock solutions and prepared in a freshly prepared culture medium.

### Electroporation Solution

Electroporation solution: this solution, which is used during electroporation, enables both more efficient delivery of the gene and avoiding high level of damage to the cells. 10-100 nM Melatonin, 1-10 mg/mL F68 and 5-50 mg/mL NaB solutions were diluted from their stock solutions and prepared in RPMI medium (Roswell Park Memorial Institute medium).

### Recovery Solution

Recovery solution: this recovery solution, which is applied on the cells right after the electroporation, enables the cells to avoid its damaging effects and recover. 10-100 nM Melatonin, 1-10 mg/mL F68 and 5-50 mg/mL NaB solutions were diluted from their stock solutions and prepared in a freshly prepared culture medium.

### Electroporation

The human tooth germ and human bone marrow stem cells, which reached sufficient concentration, were detached with IX Trypsin-EDTA, and then the cell solution was transferred to falcon tubes and precipitated by centrifugation at about 1000 rpm for 5 minutes. The cell pellet was dissolved to have 5-10 × 10⁵ cells in approximately 200µL of RPMI-1640 medium. 5 µg EGFP (Enhanced Green Fluorescent Protein) plasmid was placed thereon and then transferred to a 0.2 mm electroporation vessel. In the electroporation process, various electrical currents such as 200, 250, 300, 350, 400 and 450 mV were applied to the cells at 200-800 µF (microfarad). Immediately after the electroporation, the cells were seeded in 6-well cell culture plates and 96-well cell culture plates. The cells seeded in 6-well cell culture plates were analyzed after 48 hours in a flow cytometry device to determine the proportion of EGFP positive cells. The cells taken into 96-well cell culture plates were used in an MTS assay after approximately 24 hours, and post-electroporation cell viability was analyzed.

### Cytotoxicity Experiment

Following the electroporation process, after the cells were seeded in 96-well culture plates (Coming Glasswork, Corning, NY) at about 5000 cells/well in Dulbecco's modified Eagle's medium (DMEM) containing 10% fetal bovine serum (Invitrogen) and 1% PSA (Biological Industries, Beit Haemek, Israel), the viability levels of the cells were measured on day 2 and 4. Cell viability was measured by using 3-(4,5-di-methyl-thiazol-2-yl)-5-(3-carboxy-methoxy-phenyl)-2-(4-sulfo-phenyl)-2H-tetrazolium (MTS)-method (CellTiter96 AqueousOne Solution; Promega, Southampton, UK). 10 µl MTS solution was added onto the cells within a 100 µl growth medium and they were incubated in dark for 2 hours. After the incubation process, cell viability was observed by performing absorbance measurement via ELISA plate reader (Biotek, Winooski, VT) device at 490 nm wavelength (Figure 1-a, 1-b).

### REFERENCES

**[1].** E. Neumann, et al.; "Gene transfer into mouse lyoma cells by electroporation in high electric fields"; The EMBO Journal Vol.1 No.7 pp.841-845;1982.
**[2].** J. Gehl; "Electroporation: theory and methods, perspectives for drug delivery, gene therapy and research"; Acta Physiol Scand, 177, 437-447; 2003.
**[3].** M. E. Yalvaç et al.; "Isolation and characterization of stem cells derived from human third molar tooth germs of young adults: implications in neo-vascularization, osteo-, adipo- and neurogenesis"; The Pharmacogenomics Journal; 10, 105-113; 2010.
**[4].** A. Arnold et al.; "Comparing reagents for efficient transfection of human primary myoblasts: FuGENE 6, Effectene and ExGen 500"; DOI: 10.1111/j.1472-8206.2005.00344.x; 2006.
**[5].** I. Kerkis, et al.; "Isolation and Characterization of a Population of Immature Dental Pulp Stem Cells Expressing OCT-4 and Other Embryonic Stem Cell Markers"; DOI: 10.1159/000099617; 2007.
**[6].** Y. Tsukamoto et al.; "Mineralized Nodule Formation by Cultures of Human Dental Pulp-Derived Fibroblasts"; Archs oral. Vol. 37. NO. 12, pp. 1045-1055, 1992.
**[7].** Zhang, Walboomers et al. 2006
**[8].** E. Ferreira et al.; "Optimization of a gene electrotransfer method for mesenchymal stem cell transfection"; Gene Therapy; 15, 537-544; 2008.

## Claims

1. A method for producing an electroporation solution comprising 10-100 nM Melatonin, 1-10 mg/mL F68 and 5-50 mg/mL NaB, which comprises following steps;
- the electroporation solution is comprising 10-100 nM Melatonin, 1-10 mg/mL F68 and 5-50 mg/mL NaB by diluting stock solutions
- preparing sodium pentaborate pentahydrate stock solution with a concentration of 0.05-0.5 g/ml produced by dissolving sodium pentaborate pentahydrate (NaB) solution in a culture medium,
- preparing Pluronic F68 stock solution with a concentration of 0.05-0.5 g/ml produced by dissolving Pluronic F68 solution in cold PBS,
- preparing melatonin stock solution with a concentration of 5-15 mM produced by dissolving melatonin solution in ethanol,
- diluting said prepared stock solutions and
- mixing in the culture medium.

2. The method for producing an electroporation solution according to Claim 1, wherein RPMI (Roswell Park Memorial Institute) medium is used as the culture medium.

3. The method for producing an electroporation solution according to Claim 1, sterilizing stock solutions by being filtered through a 0.2 µm filter.

4. An electroporation process carried out by using an electroporation solution comprising 10-100 nM Melatonin, 1-10 mg/mL F68 and 5-50 mg/mL NaB and obtained by any of the method of claims 1-3, comprising the steps of
- dissolving Human Tooth Germ Stem Cells that have reached a concentration of 60% to 80% in trypsin-EDTA to obtain the cell solution,
- precipitating the cells by placing the cell solution into falcon tubes and centrifuging them,
- dissolving the precipitated cell pellet in a culture medium,
- placing EGFP plasmid in the culture medium and transferring the resulting solution to an electroporation vessel containing the electroporation solution,
- applying electric current to the cells within the scope of the electroporation process,
- seeding the cells into culture plates after electroporation,
- obtaining the cell culture to which gene transfer is performed.

5. The electroporation process according to Claim 4, wherein the cell solution transferred to falcon tubes was precipitated by centrifugation at 1000 rpm for 5 minutes.

6. The electroporation process according to Claim 4, wherein the precipitated cell pellet was dissolved to have 5-10 × 10⁵ cells in 200µL of RPMI-1640 medium.

7. The electroporation process according to Claim 4, wherein 5 µg EGFP (Enhanced Green Fluorescent Protein) plasmid was placed on the medium and then transferred to a 0.2 mm electroporation vessel.

8. The electroporation process according to Claim 4, wherein electrical currents of 200, 250, 300, 350, 400 and 450 mV were applied to the cells at 200-800 µF.

## Patentansprüche

1. Ein Verfahren zur Herstellung einer Elektroporationslösung enthaltend 10-100 nM Melatonin, 1-10 mg/mL F68 und 5-50 mg/mL NaB, wobei das Verfahren die folgenden Schritte umfasst;
- Elektroporationslösung enthält 10-100 nM Melatonin, 1-10 mg/mL F68 und 5-50 mg/mL NaB, wobei sie durch Verdünnung von Stammlösungen hergestellt wird,
- Bereitstellen einer Natriumpentaborat-Pentahydrat-Stammlösung mit einer Konzentration von 0,05-0,5 g/ml, hergestellt durch Auflösen von Natriumpentaborat-Pentahydrat (NaB)-Lösung in einem Kulturmedium,
- Bereistellen einer Pluronic F68-Stammlösung mit einer Konzentration von 0,05 - 0,5 g/ml, hergestellt durch Auflösen der Pluronic F68-Lösung in kaltem PBS,
- Bereitstellen einer Melatonin-Stammlösung mit einer Konzentration von 5-15 mM, hergestellt durch Auflösen der Melatoninlösung in Ethanol,
- Verdünnen der so bereitgestellten Stammlösungen und
- Vermischen im Nährmedium.

2. Verfahren zur Herstellung einer Elektroporationslösung nach Anspruch 1, wobei als Nährmedium RPMI-Medium (Roswell Park Memorial Institute) verwendet wird.

3. Verfahren zur Herstellung einer Elektroporationslösung nach Anspruch 1, wobei Stammlösungen durch Filtration über einen 0,2 µm Filter sterilisiert werden.

4. Ein Elektroporationsverfahren, das unter Verwendung einer Elektroporationslösung durchgeführt wird, die 10-100 nM Melatonin, 1-10 mg/mL F68 und 5-50 mg/mL NaB enthält und durch ein Verfahren nach einem der Ansprüche 1 bis 3 erhalten wurde, umfassend die folgenden Schritte:
- Auflösen der menschlichen Zahnkeim-Stammzellen bis zu einer Konzentration von 60 % bis 80 % in Trypsin-EDTA, um die Zelllösung zu erhalten,
- Ausfällen der Zellen, indem die Zelllösung in Falcon-Röhrchen gefüllt und darin zentrifugiert wird,
- Auflösen des ausgefällten Zellpellets in einem Nährmedium,
- Einbringen des EGFP-Plasmids in das Nährmedium und Überführen der entstandenen Lösung in ein Elektroporationsgefäß enthaltend die Elektroporationslösung,
- Anlegen von elektrischem Strom an die Zellen im Rahmen des Elektroporationsverfahrens,
- Aussäen der Zellen in Kulturplatten nach der Elektroporation,
- Gewinnung der Zellkultur, auf die der Gentransfer erfolgen soll.

5. Elektroporationsverfahren nach Anspruch 4, wobei die in Falcon-Röhrchen überführte Zelllösung durch Zentrifugation bei 1000 U/min für 5 Minuten ausgefällt wird.

6. Elektroporationsverfahren nach Anspruch 4, wobei das ausgefällte Zellpellet so aufgelöst wird, dass es 5-10 × 10⁵ Zellen in 200 µL RPMI-1640-Medium enthält.

7. Elektroporationsverfahren nach Anspruch 4, wobei 5 µg EGFP (Enhanced Green Fluorescent Protein) Plasmid auf das Medium gegeben und dann in ein 0,2 mm Elektroporationsgefäß überführt werden.

8. Elektroporationsverfahren nach Anspruch 4, wobei elektrische Spannungen von 200, 250, 300, 350, 400 und 450 mV bei 200-800 µF an die Zellen angelegt werden.

## Revendications

1. Méthode de production d'une solution d'électroporation comprenant 10-100 nM de mélatonine, 1-10 mg/mL de F68 et 5-50 mg/mL de NaB, comprenant les étapes suivantes :
- la solution d'électroporation comprenant 10-100 nM de mélatonine, 1-10 mg/mL F68 et 5-50 mg/mL NaB par dilution des solutions mères,
- préparation de solution mère de pentaborate de sodium pentahydraté d'une concentration de 0,05-0,5 g/mL obtenue par dissolution d'une solution de pentaborate de sodium pentahydraté (NaB) dans un milieu de culture,
- préparation de solution mère de Pluronic F68 ayant une concentration de 0,05-0,5 g/mL obtenue par dissolution de la solution mère de Pluronic F68 dans du PBS froid,
- préparation de solution mère ayant une concentration de 5-15 mM obtenue par dissolution de la solution de mélatonine dans de l'éthanol,
- dilution de lesdites solutions mères préparées et
- mélange des mêmes au milieu de culture.

2. Méthode de production d'une solution d'électroporation selon la revendication 1, dans laquelle le milieu RPMI (Roswell Park Memorial Institute) est utilisé comme milieu de culture.

3. Méthode de production d'une solution d'électroporation selon la revendication 1, stérilisant les solutions de base en les filtrant à travers un filtre de 0,2 µm.

4. Procédé d'électroporation réalisé à l'aide d'une solution d'électroporation comprenant 10-100 nM de mélatonine, 1-10 mg/mL de F68 et 5-50 mg/mL de NaB et obtenue par l'une quelconque des méthodes des revendications 1 à 3, comprenant les étapes suivantes :
- dissolution des Cellules Souches Germinales Dentaires Humaines ayant atteint une concentration de 60 à 80% dans de la trypsine-EDTA afin d'obtenir la solution cellulaire,
- précipitation des cellules en plaçant la solution cellulaire dans des tubes falcon et en les centrifugeant,
- dissolution de la plaquette cellulaire précipitée dans un milieu de culture,
- placement du plasmide EGFP dans le milieu de culture et transfert de la solution résultante dans un récipient d'électroporation contenant la solution d'électroporation,
- l'application d'un courant électrique aux cellules dans le cadre du processus d'électroporation,
- l'ensemencement des cellules dans des plaques de culture après l'électroporation,
- l'obtention de la culture cellulaire dans laquelle le transfert de gènes est effectué.

5. Procédé d'électroporation selon la revendication 4, dans lequel la solution cellulaire transférée dans des tubes falcon a été précipitée par centrifugation à 1000 rpm pendant 5 minutes.

6. Le procédé d'électroporation selon la revendication 4, dans lequel le culot cellulaire précipité a été dissous pour avoir 5-10 × 10⁵ cellules dans 200 µL de milieu RPMI-1640.

7. Procédé d'électroporation selon la revendication 4, dans lequel 5 µg de plasmide EGFP (Enhanced Green Fluorescent Protein) a été placé sur le milieu, puis transféré dans un récipient d'électroporation de 0,2 mm.

8. Procédé d'électroporation selon la revendication 4, dans lequel des courants électriques de 200, 250, 300, 350, 400 et 450 mV ont été appliqués aux cellules à 200-800 µF.
